Europäisches Patentamt

European Patent Office

Office européen des brevets

(19)

(11) **EP 0 691 540 B1**

(12) **FASCICULE DE BREVET EUROPEEN**

(45) Date de publication et mention
de la délivrance du brevet:
**26.02.2003 Bulletin 2003/09**

(51) Int Cl.[7]: **G01N 33/24**, G01N 31/12

(21) Numéro de dépôt: **95401531.9**

(22) Date de dépôt: **27.06.1995**

(54) **Méthode améliorée permettant l'évaluation rapide d'au moins une caractéristique pétrolière d'un échantillon de roche, et application à un gisement comportant des huiles lourdes**

Verbessertes Verfahren zur schnellen Auswertung von mindestens einer petrologischen Eigenschaft einer Steinprobe und Anwendung desselben bei Schweröl enthaltenden Schichten

Improved method for the rapid determination of at least one petrological characteristic of a sample of rock and its application to strata containing heavy oil

(84) Etats contractants désignés:
**DE GB IT NL**

(30) Priorité: **05.07.1994 FR 9408383**

(43) Date de publication de la demande:
**10.01.1996 Bulletin 1996/02**

(73) Titulaire: **INSTITUT FRANCAIS DU PETROLE
92500 Rueil Malmaison (FR)**

(72) Inventeurs:
• **Trabelsi, Khomsi
F-78100 Conflans Sainte Honorine (FR)**

• **Espitalie, Jean
F-78110 Le Vesinet (FR)**

(56) Documents cités:
**EP-A- 0 026 012     US-A- 4 229 181
US-A- 4 352 673     US-A- 4 629 702**

• **PETROLE ET TECHNIQUES, no. 283, Octobre 1981 PARIS FR, pages 99-103, ESPITALIE J ET AL 'Méthode rapide d'analyse géochimique des déblais en suivi de forage'**
• **TRAC, TRENDS IN ANALYTICAL CHEMISTRY, vol. 1, no. 10, Juin 1982 CAMBRIDGE GB, pages 237-241, PHILP R P 'Application of pyrolysis-GC and pyrolysis-GC-MS to fossil fuel research'**

**Description**

La présente invention concerne une méthode pour déterminer au moins une caractéristique pétrolière d'une roche sédimentaire potentiellement ou réellement productrice d'hydrocarbures.

Pour une meilleure compréhension, on rappelle que:

- les composés du pétrole, principalement les hydrocarbures extractibles par les solvants organiques, se forment à partir de la matière organique insoluble (kérogène) sous l'action de l'élévation de température et de pression qui résultent de l'enfouissement des sédiments dans un bassin sédimentaire,
- la connaissance de la matière organique soluble (pétrole) d'une part, et de la matière organique insoluble (kérogène) d'autre part, est d'un grand intérêt en exploration et en exploitation pétrolière. Il est connu en effet que:
- la quantité de pétrole formée dans les sédiments augmente régulièrement avec la profondeur d'enfouissement. De ce fait, il est possible d'apprécier le degré d'évolution de la matière organique contenue dans ces sédiments et, plus particulièrement, l'intervalle d'évolution qui correspond à la phase principale de formation d'huile;
- ce sont ces pétroles qui, dans certaines conditions, vont être expulsés de la roche mère dans laquelle ils se sont formés pour donner les accumulations de pétrole dans les roches réservoirs;
- la nature de la matière organique insoluble contenue dans les roches conditionne le potentiel pétroligène de ces roches, c'est-à-dire leur aptitude plus ou moins grande à produire du pétrole.

Il apparaît alors que l'analyse systématique de ces composées organiques permet de distinguer, parmi les sédiments géologiques, ceux qui présentent le plus d'intérêt pour le géologue :

- sédiments ayant donné naissance au pétrole (roches-mères),
- sédiments où s'est accumulé ce pétrole (roches réservoirs).

La connaissance de tels renseignements permet aux foreurs de n'effectuer les opérations de carottage ou d'essai de réservoir, opérations longues, coûteuses et périlleuses, qu'à bon escient, surtout lorsque ces résultats peuvent être acquis simultanément aux opérations de forage.

On connaît la technique ROCK-EVAL développée par la demanderesse et décrite notamment dans les documents US-3953171, US-4153415, US-4229181, US-4352673 et US-4519983. Cette méthode qui est rapide, pratiquement automatique, peut être appropriée à la caractérisation des roches et des hydrocarbures qu'elles contiennent.

Cependant, la technique ROCK-EVAL existante a été développée dans le but de caractériser les roches-mères pour lesquelles elle donne de bonnes informations sur la matière organique contenue dans des roches-mères. Mais cette méthode n'est pas totalement satisfaisante pour caractériser les hydrocarbures contenus dans une roche réservoir, notamment pour identifier les dépôts de "tar-mat", appellation consacrée des composés lourds du pétrole (résines et asphaltènes) qui se déposent dans certains niveaux des réservoirs. Ces tar-mats peuvent présenter des inconvénients majeur pour la production, notamment en créant des barrières de perméabilité verticale ou parfois des venues d'eau dans les horizons productifs. La localisation de ces niveaux de tar-mat et leur extension latérale à l'échelle du réservoir sont donc très importantes pour, d'une part, comprendre les processus d'accumulation des hydrocarbures et d'autre part pour optimiser la récupération des hydrocarbures présents.

Ainsi, la méthode selon la présente invention, est un perfectionnement de la technique ROCK-EVAL, permettant de caractériser les différentes coupes d'hydrocarbures, de la plus légère à la plus lourde, contenues dans une roche réservoir.

La présente invention concerne une méthode améliorée permettant l'évaluation rapide d'au moins une caractéristique pétrolière d'un échantillon provenant de sédiments géologiques, méthode dans laquelle ledit échantillon est chauffé en atmosphère non oxydante selon plusieurs étapes d'élévation de température. La méthode comporte les étapes suivantes:

a) on élève rapidement la température dudit échantillon à une première valeur de température inférieure à 200°C et pendant une durée déterminée,

b) à partir de la première valeur de température, on élève la température de l'échantillon jusqu'à une seconde valeur de température selon un gradient de température compris entre 1 et 12°C/minute,

c) on élève la température de l'échantillon de la seconde valeur à une troisième valeur de température au plus égale à 800°C, préférentiellement inférieure à 600°C,

d) on détermine trois grandeurs $S_{1r}$, $S_{2a}$ et $S_{2b}$ représentatives de la quantité des composée hydrocarbonés contenus dans ledit échantillon,

e) on déduit de ces trois grandeurs au moins une caractéristique pétrolière dudit échantillon.

La grandeur $S_{1r}$ peut être représentative des hydrocarbures légers, tels ceux pour lesquels le nombre de carbone est inférieur à environ 15, $S_{2a}$ peut être représentative des hydrocarbures plus lourds, tels ceux pour lesquels le nombre de carbone est compris entre environ 15 et 40, $S_{2b}$ peut être représentative des composés hydrocarbonés lourds, tels ceux pour lesquels le nombre de carbone est supérieur à environ 40.

Après l'élévation de la température à la troisième valeur, on peut brûler les résidus dudit échantillon et on peut déterminer une quatrième valeur $R_C$ représentative de la quantité de carbone organique résiduel après pyrolyse.

On peut évaluer la quantité Q de produits lourds selon la formule suivante:

$$Q = S_{2b} + 10R_C/0,9$$

On peut distinguer certains hydrocarbures de l'échantillon en calculant un indice de production IP selon la formule suivante:

$$IP = (S_{1r} + S_{2a})/(S_{1r} + S_{2a} + S_{2b})$$

et pour une valeur de μ comprise entre 0 et 1, si IP > μ l'échantillon peut contenir des résines et/ou asphaltènes et si IP <μ l'échantillon peut contenir du kérogène.

On peut prendre pour μ une valeur voisine de 0,4.

Dans une variante de la méthode dans laquelle ledit échantillon est un morceau de roche réservoir:

- on peut calculer une grandeur D pour plusieurs échantillons de roche réservoir à partir de l'une au moins des grandeurs $S_{1r}$, $S_{2a}$, $S_{2b}$ et Q,
- on peut mesurer le degré API du pétrole produit par lesdites roches réservoirs desdits échantillons,
- on peut déterminer une fonction approchée f reliant le degré API mesuré à D,
- on peut déterminer une valeur approchée du degré API du pétrole en place dans une autre roche réservoir en prélevant au moins un échantillon de ladite autre roche, en calculant la grandeur D correspondante et en utilisant directement la fonction f.

Selon une autre variante de la méthode dans laquelle ledit échantillon est une portion de pétrole produit:

- on peut calculer une grandeur D pour plusieurs échantillons provenant de différents réservoirs à partir de l'une au moins des grandeurs $S_{1r}$, $S_{2a}$, $S_{2b}$ et Q,
- on peut mesurer le degré API desdits échantillons,
- on peut déterminer une fonction approchée f1 reliant le degré API mesuré à D,
- on peut déterminer une valeur approchée du degré API d'un pétrole d'une autre provenance, en calculant la grandeur D correspondante à un échantillon dudit pétrole d'une autre provenance et en utilisant directement la fonction f1.

La grandeur D peut être égale à $S_{2b}/(S_{1r} + S_{2a})$.

La grandeur D peut être égale à $S_{1r}/(S_{1r} + S_{2a} + Q)$.

La grandeur D peut être égale à $(S_{1r} + S_{2a})/(S_{1r} + S_{2a} + Q)$

La fonction f ou f1 peut avoir la forme suivante:

$$D = a \times 10^{be^{-\alpha x}} + C$$

x étant le degré API; a est compris entre 0,11 et 0,12 et préférentiellement égal à 0,11482; b est compris entre 1,7 et 2 et préférentiellement égal à 1,83; c voisin de zéro; α voisin de 2.

L'invention sera mieux comprise et ses avantages apparaîtront plus clairement à la lecture de la description d'exemples, nullement limitatifs, illustrés par les figures annexées, parmi lesquelles:

- la figure 1 représente schématiquement la composition d'une roche pétrolière,
- la figure 2 montre les étapes de chauffe de l'échantillon en fonction du temps,
- la figure 3 montre la forme du signal S,
- la figure 4 illustre un exemple d'application,
- la figure 5 montre un système de mise en oeuvre de la méthode,

- la figure 6 illustre un exemple d'affichage de certaines grandeurs caractéristiques,
- la figure 7 illustre une variante selon l'invention.

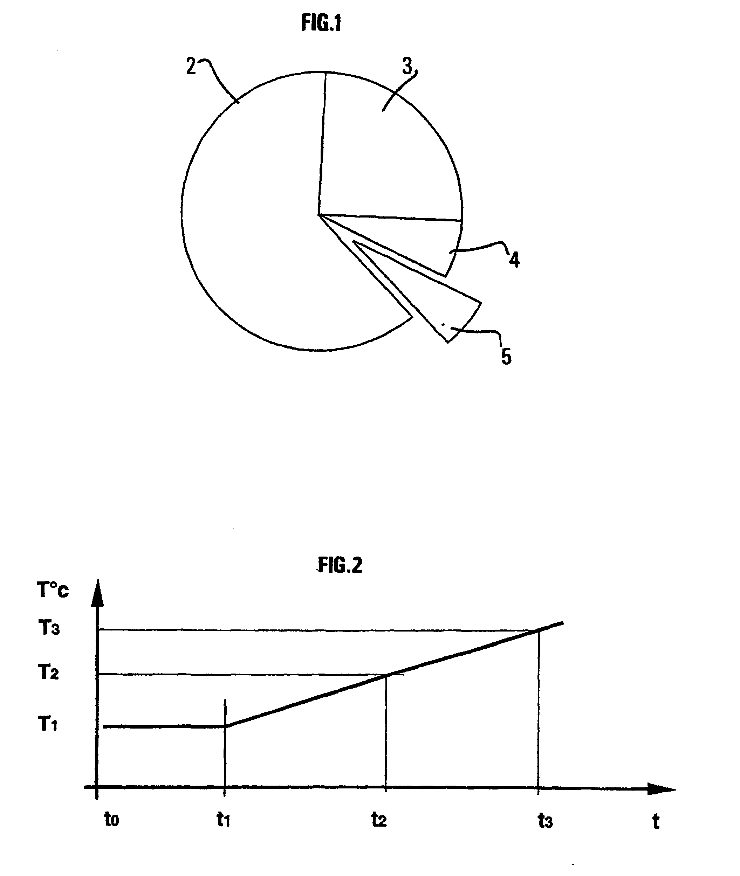

La figure 1 illustre schématiquement la composition d'un échantillon de roche sédimentaire, par exemple un débris de forage "cuttings" provenant d'une formation géologique traversée par un forage en cours d'exécution.

La zone 2 représente la quantité de matériau minéral, la zone 3 représente la quantité d'hydrocarbures dits légers. Généralement, le nombre de carbone de ces hydrocarbures est inférieur à 15.

La zone 4 représente la quantité d'hydrocarbures intermédiaires entre les hydrocarbures légers et les hydrocarbures lourds. Généralement on peut les classer entre $C_{15}$ et $C_{40}$.

La zone 5 représente la quantité de composés hydrocarbonés lourds, résines, asphaltènes et/ou de kérogène, généralement supérieur à $C_{40}$.

La méthode selon la présente invention va permettre de distinguer et de quantifier les constituants organiques contenus dans un échantillon de roche et en particulier, différencier les résines et les asphaltènes (NSO) du kérogène. Ainsi, au cours d'un forage à travers une formation géologique productrice ou potentiellement productrice, on pourra prélever des échantillons de la formation en fonction de la profondeur. Ces échantillons, une fois caractérisés par le moyen de la présente méthode, vont permettre l'établissement de "logs" ou représentation graphique des couches de terrains différenciées par la quantité et/ou la nature des hydrocarbures qu'elles contiennent.

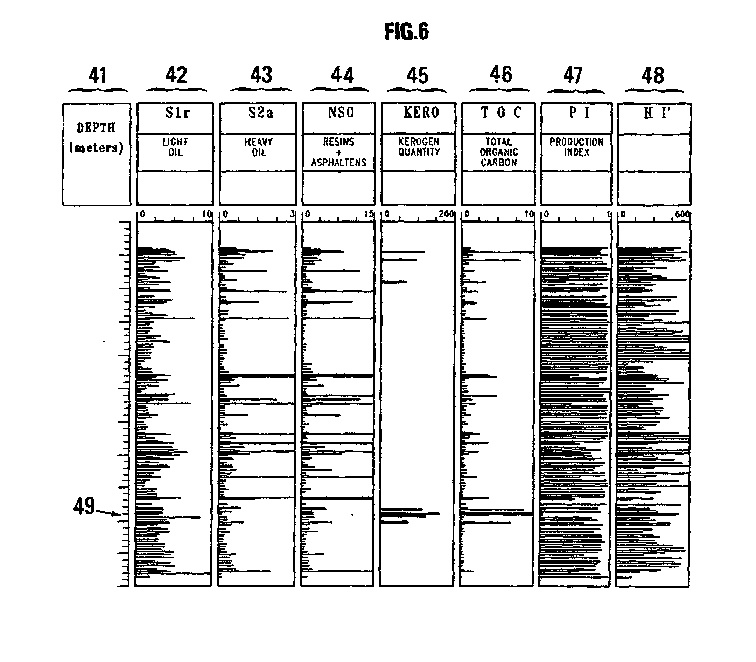

On ne décrira pas ici les moyens de pyrolyse et d'oxydation de résidus de pyrolyse, il suffira de se reporter aux documents précités qui décrivent les moyens utilisés pour appliquer la présente invention. On rappelle cependant que le four à pyrolyse coopère avec un dispositif de détection et de mesure de la quantité d'hydrocarbures de l'échantillon pyrolysé. Le dispositif de détection spécifique (figure 5) comprend, par exemple, un détecteur du type à ionisation de flamme, d'utilisation classique dans les analyses par chromatographie en phase gazeuse. Le détecteur délivre un signal S représentatif des quantités de produits hydrocarbonés mesurés. Ce signal peut être transmis à des moyens de calcul, de mémorisation et d'affichage dans lesquels un logiciel spécifique calcule, affiche et mémorise les différents paramètres représentatifs des caractéristiques des hydrocarbures en présence.

Pour mesurer le carbone résiduel de pyrolyse, on pourra utiliser les moyens décrits dans les documents US-4352673 et US-4519983. Des moyens automatiques peuvent transférer des résidus de pyrolyse dans un four spécifique d'oxydation, ou dans une variante, on pourra utiliser un four unique pour la pyrolyse en atmosphère inerte et pour l'oxydation.

La figure 2 représente les séquences de température de l'opération de pyrolyse. Au temps $t_0$, l'échantillon est introduit dans le four chauffé à la température initiale $T_1$. Cette valeur de température initiale est inférieure à 200°C, préférentiellement proche de 180°C. La durée $t_1$-$t_0$ de cette première étape de chauffe est par exemple d'environ 15 minutes. A partir du temps $t_1$ débute une phase de pyrolyse programmée dans laquelle la montée en température est impérativement inférieure à 12°C/minute jusqu'au temps $t_2$ correspondant à la température $T_2$. La valeur de cette température est d'environ 370°C qui correspond sensiblement à la fin de la phase de thermovaporisation de certains hydrocarbures et le début de la phase de craquage par pyrolyse des composés lourds.

D'une manière préférentielle, la pyrolyse se poursuit après le temps $t_2$ de façon à atteindre la température de $T_3$, au plus égale à 800°C et préférentiellement environ de 600°C au temps $t_3$. La montée programmée en température peut être la même que celle entre les températures $T_1$ $T_2$, c'est-à-dire inférieure à 12°C/minute, ce qui simplifie la méthode. Mais dans certains cas, il pourra être possible d'effectuer l'étape de craquage des hydrocarbures lourds selon une montée en température plus rapide que 12°C/minute ou différente du gradient de température entre $T_1$ et $T_2$.

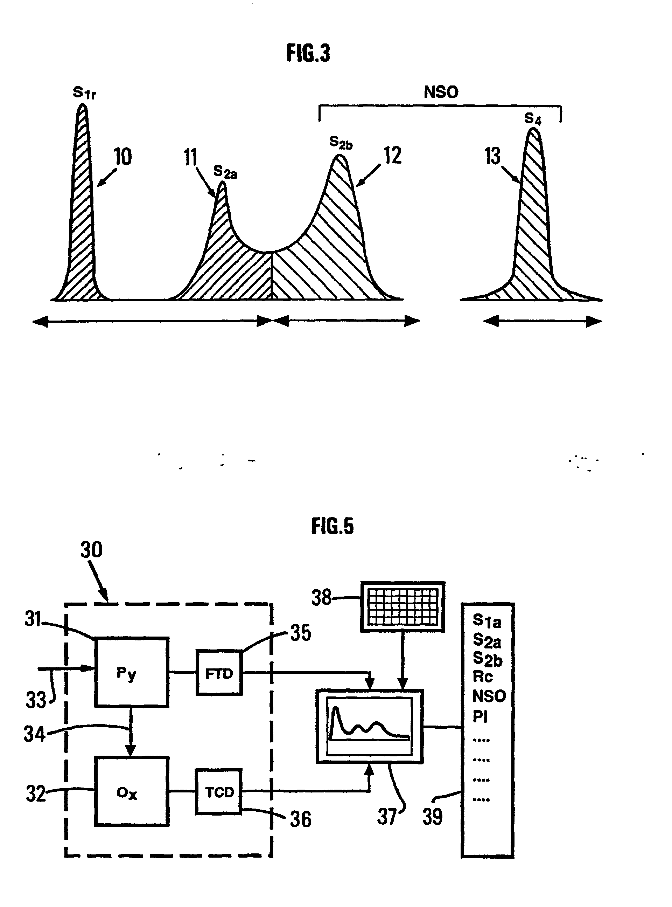

Sur la figure 3, on peut voir une illustration des grandeurs représentatives des composés hydrocarbonés contenus dans une roche réservoir imprégnée selon la composition représentée sur la figure 1.

On constate que dans les conditions de chauffage décrits figure 2, une roche imprégnée contenant des produits lourds (résines, asphaltènes et/ou kérogènes) donne en cours de pyrolyse un premier pic $S_{1r}$, un pic $S_{2a}$ et un pic $S_{2b}$, respectivement référencés 10, 11 et 12.

Les pics $S_{1r}$ et $S_{2a}$ (10, 11) correspondent à la thermovaporisation des hydrocarbures inférieurs à environ C40 présents dans l'échantillon de réservoir, alors que le pic $S_{2b}$ (12) correspond aux composés hydrocarbonés issus du craquage par pyrolyse des résines et des asphaltènes et/ou du kérogène qui constituent les composés lourds.

Bien entendu, pour une roche réservoir imprégnée qui ne contient pas de résines et asphaltènes (NSO), la caractérisation ne comportera qu'un pic $S_{1r}$ (10) et un pic $S_{2a}$ (11).

Dans le cas d'une roche imprégnée qui contient des quantités de NSO, un pic $S_{2b}$ apparaît dont l'amplitude sera fonction desdites quantités.

Des moyens de calculs déterminent les quantités respectives des différents types d'hydrocarbures (hydrocarbures légers, hydrocarbures lourds, totalité des résines et asphaltènes et/ou du kérogène) en fonction de la forme, de l'amplitude desdits pics et du carbone organique restant après pyrolyse.

En effet, la pyrolyse des produits lourds, notamment les asphaltènes, s'accompagne toujours de la formation de coke. Le coke est ensuite brûlé dans le second four d'oxydation de l'appareillage ROCK-EVAL et le $CO_2$ produit

est mesuré par un pic représentatif d'une grandeur caractéristique $S_4$ (13). On détermine ainsi le pourcentage de carbone organique résiduel dans l'échantillon, dénommé carbone organique résiduel $R_C$ restant après pyrolyse.

Des analyses élémentaires effectuées sur le coke montrent que sa teneur en carbone organique est en moyenne de 90%.

On détermine pour chaque échantillon pyrolysé la quantité totale Q de produits lourds contenus dans l'échantillon en ajoutant à la quantité de composés représentés par le pic $S_{2b}$ la quantité de composés hydrocarbonés que représente le coke formé, ceux-ci étant obtenus à partir du carbone résiduel $R_C$ déterminé par oxydation dans le ROCK-EVAL. La quantité Q est alors égale à

$$Q = S_{2b} + 10R_C/0,9$$

Q et $S_{2b}$ étant exprimés en milligramme par gramme.

Q sera appelée NSO lorsqu'il s'agit principalement de résines et asphaltènes.

Q pourra être appelée KERO lorsqu'il s'agit principalement de kérogène.

C'est à partir de cette quantité Q ou NSO que l'on pourra juger de l'importance des "tars" déposés dans le réservoir.

Il est courant que des débris de matière organique insoluble (par exemple des débris charbonneux dans les réservoirs gréseux) ou que des intercalations de roche-mère soient présentes dans les réservoirs. Dans ces conditions, la matière organique insoluble de ces débris ou de ces intercalations de roche-mère peut être confondue, en pyrolyse, avec la présence de "tars". En effet, les composés issus du craquage thermique de la matière organique insoluble apparaissent au niveau du pic $S_{2b}$ avec formation de coke.

Pour différencier le kérogène des "tars", on utilise les valeurs d'un index de production PI calculé selon la formule suivante:

$$PI = (S_{1r} + S_{2a})/(S_{1r} + S_{2a} + S_{2b})$$

Une valeur faible de PI indique que l'on est en présence de kérogène, on considère alors que $S_{2b}$ représente la partie pyrolysable du kérogène et non uniquement celles des résines et asphaltènes. Dans ce cas, la quantité Q calculée représentera la quantité de kérogène.

Un contrôle de la présente méthode a été effectué par extraction de composés lourds par solvant. On a pu vérifier que la valeur de 0,4 pour PI est une bonne coupure (cut-off) pour différencier le kérogène des composés extractibles lourds (résines et asphaltènes). Cette valeur peut être éventuellement précisée par un étalonnage préalable des échantillons étudiés.

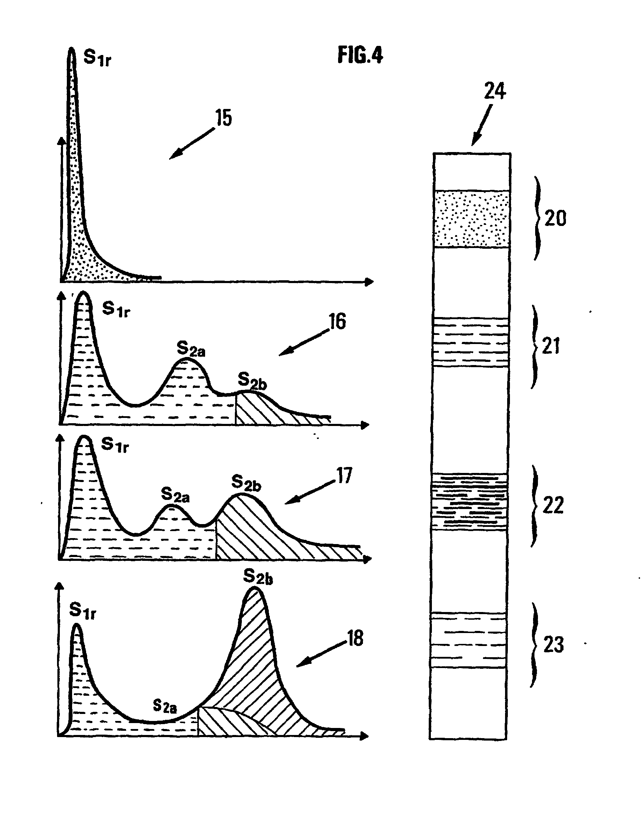

La figure 4 montre quatre exemples de pyrolyse sur des échantillons de roche contenant différents types de matières organiques.

La référence 24 indique un figuré de la lithologie, dans laquelle la référence 20 représente une formation réservoir contenant du gaz, la référence 21 représente une roche réservoir contenant des hydrocarbures liquides, la référence 22 représente une roche réservoir contenant des résines et asphaltènes ou "tars", la référence 23 représente une intercalation de roche-mère contenant du kérogène (matière organique insoluble).

Des échantillons prélevés dans chacune des quatre zones a permis l'obtention des graphiques 15, 16, 17 et 18 représentant les grandeurs associées aux différents hydrocarbures, selon la présente invention.

Le graphe 15 montre la seule présence d'un pic $S_{1r}$ représentatif d'un hydrocarbure très léger comme du gaz.

Le graphe 16 montre la présence du pic $S_{1r}$ et du pic $S_{2a}$ significative d'un hydrocarbure liquide. Le pic $S_{2b}$ peut apparaître ou ne pas apparaître en fonction du taux de résines et asphaltènes que peut contenir la présente roche réservoir 21. La valeur de PI est supérieure à 0,4.

Le graphe 17 montre la présence d'un pic $S_{2b}$ relativement important par rapport aux pics $S_{1r}$ et $S_{2a}$. Cette représentation permet de reconnaître dans la roche réservoir du type 21 une intercalation d'un niveau de "tars" ou résines et asphaltènes, référencée 22.

Le graphe 18 correspond principalement à la présence des deux seuls pics, $S_{1r}$ et $S_{2b}$. Le pic $S_{2a}$, généralement petit, et les composés lourds (résines et asphaltènes) sont masqués dans le pic $S_{2b}$. La roche 23 est identifiée comme étant une roche-mère, le pic $S_{2b}$ étant représentatif des hydrocarbures issus du craquage du kérogène. La valeur du PI est généralement inférieure à 0,4.

La figure 5 montre une schématisation des moyens de mise en oeuvre de la méthode selon l'invention.

La référence 30 indique le système comprenant des moyens de pyrolyse 31, des moyens d'oxydation 32, chacun associés respectivement à des moyens de détection 35 des hydrocarbures (par exemple un détecteur à ionisation de flamme FID) et à des moyens de détection 36 (par exemple un détecteur à conductivité thermique TCD) du carbone résiduel oxydé dans le four 32.

La flèche référencée 33 symbolise les moyens d'introduction de l'échantillon dans le système.

La référence 37 montre des moyens de calculs, de mémorisation, d'affichage des grandeurs détectées par ledit système. Il sera également possible que les moyens 37 pilotent certains paramètres de fonctionnement du système 30.

Un clavier 38 permet d'introduire des commandes dans les moyens 37.

La référence 39 montre une liste des grandeurs caractéristiques que peuvent fournir les moyens de calculs 37. Un exemple d'enregistrement des différentes grandeurs est illustré figure 6.

La figure 6 montre une représentation de certaines grandeurs caractéristiques des roches traversées par un forage entre 1300 m et 2500 m. La colonne 41 donne l'échelle des profondeurs en mètre. La colonne 42 donne la quantité d'hydrocarbures légers, à partir du pic $S_{1r}$, en milligramme par gramme d'échantillon. La colonne 43 donne la quantité d'hydrocarbures plus lourds, à partir du pic $S_{2a}$, dans la même unité. La colonne 44 donne la quantité de NSO (résines et/ou asphaltènes) obtenue à l'aide du pic $S_{2b}$ et du pic $S_4$. La colonne 45 indique et donne la quantité KERO de kérogène. La colonne 46 donne la quantité totale de carbone organique contenu dans l'échantillon, en pourcentage par rapport au poids d'échantillon. La colonne 47 donne la valeur de l'index de production calculée à partir des pics $S_{1r}$, $S_{2a}$ et $S_{2b}$. En fonction d'une valeur de coupure donnée ("cut-off"), par exemple 0,4, le taux de NSO calculé est identifié comme étant du kérogène (colonne 5) ou du NSO vrai (colonne 44). On peut remarquer que les échantillons de la profondeur référencée 49 ont des indices de production faibles, de l'ordre de 0,1 et qu'il est indiqué la présence de kérogène. On peut déduire qu'à ce niveau les sédiments contiennent des intercalations de roche-mère. La colonne 48 donne un indice d'hydrogène.

Avec la méthode selon l'invention, il est avantageux également d'utiliser les grandeurs $S_{1r}$, $S_{2a}$, $S_{2b}$ et $R_C$ obtenus sur des échantillons de roches traversées par un forage, pour évaluer le degré API des hydrocarbures contenus dans les roches réservoirs. La connaissance du degré API est très intéressante car le degré API est la grandeur couramment utilisée pour déterminer notamment la mobilité des hydrocarbures. Il est connu que des hydrocarbures liquides ayant un degré API inférieur à 14 ne peuvent être produits avec des méthodes conventionnelles. Aussi, dans certains réservoirs, la connaissance du degré API acquise en même temps que l'on fore le réservoir, permet d'effectuer les tests de production aux niveaux où la production sera effective.

De plus, la méthode peut être appliquée à la détermination du degré API d'un pétrole produit sans avoir à effectuer les mesures conventionnelles de laboratoire pour mesurer le degré API.

Pour pouvoir déterminer le degré API, il faut au préalable déterminer une ou plusieurs lois ou fonctions qui relient le degré API à au moins une des grandeurs caractéristiques $S_{1r}$, $S_{2a}$, $S_{2b}$ et éventuellement $\mathbf{Q = S_{2b} + 10R_C/0,9}$. Pour cela, on collecte un certain nombre d'échantillons de roches réservoirs et de pétrole ayant été contenu par ces roches réservoirs. On mesure par des méthodes traditionnelles de laboratoire le degré API du pétrole d'une part, et on pyrolyse l'échantillon correspondant selon la présente méthode d'autre part.

Il a été observé par la demanderesse qu'une grandeur D égale à l'une des formules suivantes:

$$D= S_{2b}/(S_{1r} + S_{2a})$$

$$D=S_{1r}/(S_{1r}+S_{2a}+Q)$$

$$D=(S_{1r}+S_{2a})/(S_{1r}+S_{2a} +Q),$$

peut être reliée au degré API par une fonction f, par exemple de la forme:

$$S_{1r}/(S_{1r}+S_{2a}+Q)=f(x) = a \times 10^{be^{\alpha x}} + C$$

$$S_{2b}/(S_{1r} + S_{2a}) = f(x) = d - h \times Logx$$

$$S_{2b}/(S_{1r}+S_{2a})=f(x) = g-he^{-\alpha x} +j^{-\beta x^2}$$

ou

$$(S_{1r}+S_{2a})/(S_{1r}+S_{2a}+Q) = k+1 \times e^{-\delta x}$$

x étant le degré API; a est compris entre 0,11 et 0,12 et préférentiellement égal à 0,11482; b est compris entre 1,7 et 2 et préférentiellement égal à 1,83; c voisin de zéro; d compris entre 2,1 et 2,3 et préférentiellement égal à 2,2273; h compris entre 1,3 et 1,4 et préférentiellement égal à 1,341; g compris entre 1,4 et 1,5 et préférentiellement égal à 1,4386; h compris entre 5,6 et 5,8 et préférentiellement égal à 5,7721; j compris entre 5,8 et 5,9 et préférentiellement égal à 5,8571; $\alpha$ voisin de 2 et $\beta$ voisin de 4.

L'obtention de la fonction approchée f se fait selon les méthodes mathématiques connues pour déterminer une fonction à partir d'un certain nombre de points.

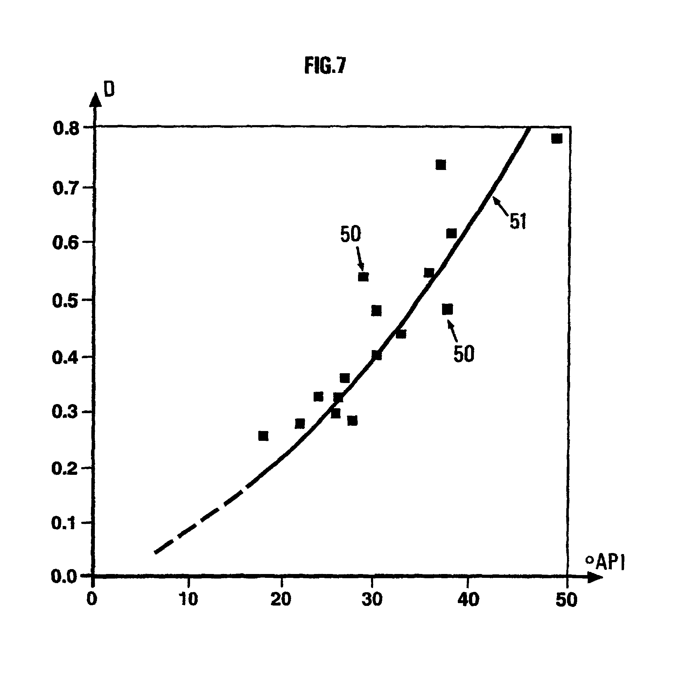

La figure 7 montre un ensemble de points 50 placés sur un graphe où le degré API est en abscisse et la grandeur $D = S_{1r}/(S_{1r} + S_{2a} + Q)$ en ordonnée.

Les coordonnées des points 50 ont été obtenues comme décrit plus haut, par mesures sur des échantillons de pétrole et de roches réservoirs de provenances différentes. En effet, on ne s'est pas limité à un seul champ pétrolier, mais il a été collecté des échantillons de différents champs, de différentes profondeurs et de différents types de réservoirs.

La courbe 51 représentant la fonction f peut ainsi être utilisée sans distinction.

Il est simple, une fois obtenue une fonction f, de l'incorporer en mémoire, par exemple dans les moyens de calculs 37 de la figure 5. Un programme de calcul peut déterminer D et en appliquant la fonction f on obtient directement par calcul une valeur approchée du degré API des hydrocarbures qui sont ou qui ont été présents dans les sédiments d'où provient l'échantillon de roche.

En variante, il sera possible de déterminer plusieurs fonctions reliant le degré API à différentes grandeurs D calculées à partir de $S_{1r}$, $S_{2a}$, $S_{2b}$ et $R_C$. On peut choisir, soit manuellement, soit automatiquement, la fonction pour obtenir le degré API, par exemple compte tenu de situations ou de circonstances déjà connues.

Une autre variante de la méthode est de déterminer une fonction f1, généralement différente de f, à partir d'un échantillonnage de pétrole de provenances diverses. Pour chaque échantillon de pétrole, on mesure le degré API selon les méthodes conventionnelles de laboratoire et on pyrolyse une faible quantité de cet échantillon selon la méthode de la présente invention afin de calculer D selon l'une des formules données ci-dessus. Une fois la fonction f1 obtenue, elle sera utilisée pour déterminer le degré API d'un autre pétrole en déterminant pour celui-ci une valeur de D en pyrolysant l'échantillon de pétrole selon la présente méthode et en appliquant directement la fonction f1 préalablement obtenue.

Ainsi, cette variante remplace les mesures conventionnelles de degré API lorsque l'on est en mesure d'appliquer la méthode selon l'invention et lorsque la détermination de la fonction f ou f1 est faite. La présente méthode est, dans certains cas, plus rapide et plus facile que les méthodes conventionnelles.

**Revendications**

1. Méthode améliorée permettant l'évaluation rapide d'au moins une caractéristique pétrolière d'un échantillon provenant de sédiments géologiques, méthode dans laquelle ledit échantillon est chauffé en atmosphère non oxydante selon plusieurs étapes d'élévation de température, **caractérisée en ce que** ladite méthode comporte les étapes suivantes:

a) on élève rapidement la température dudit échantillon à une première valeur de température inférieure à 200°C et pendant une durée déterminée,
b) à partir de la première valeur de température, on élève la température de l'échantillon jusqu'à une seconde valeur de température d'environ 370°C selon un gradient de température compris entre 1 ci 12°C/minute,
c) on élève la température de l'échantillon de la seconde valeur à une troisième valeur de température au plus égale à 800°C,
d) on détermine respectivement au cours desdites étapes a, b et c, trois grandeurs $S_{1r}$, $S_{2a}$ et $S_{2b}$ représentatives de la quantité des composés hydrocarbonés contenus dans ledit échantillon, **en ce que** la grandeur $S_{1r}$ est représentative des hydrocarbures légers, tels ceux pour lesquels le nombre de carbone est inférieur à environ 15, **en ce que** $S_{2a}$ est représentative des hydrocarbures plus lourds, tels ceux pour lesquels le nombre de carbone est compris entre environ 15 et 40, $S_{2b}$, est représentative des composés hydrocarbonés lourds (résines, asphaltènes et/ou kérogène), tels ceux pour lesquels le nombre de carbone est supérieur à environ 40.

2. Méthode selon la revendication 1, **caractérisée en ce qu'**après l'élévation de la température à la troisième valeur, on brûle les résidus dudit échantillon et **en ce que** l'on détermine une quatrième valeur $R_C$ représentative de la quantité de carbone organique résiduel après pyrolyse.

3. Méthode selon l'une des revendications 1 à 2, **caractérisée en ce que** l'on évalue une quantité Q de produits lourds selon la formule suivante:

$$Q= S_{2b} + 10R_C/0{,}9$$

(Q et S2b en milligramme/gramme)

4. Méthode selon l'une des revendications 1 à 3, **caractérisée en ce que** l'on distingue certains hydrocarbures de l'échantillon en calculant un indice de production IP selon la formule suivante:

$$IP = (S_{1r} + S_{2a})/(S_{1r} + S_{2a} + S_{2b})$$

et **en ce que** pour IP > $\mu$, $\mu$ étant compris entre 0 et 1, l'échantillon contient des résines et/ou asphaltènes et pour IP < $\mu$, l'échantillon contient du kérogène.

5. Méthode selon la revendication 4, **caractérisée en ce que** l'on prend pour une valeur voisine de 0,4.

6. Méthode selon l'une des revendications 1 à 5, dans laquelle ledit échantillon est un morceau de roche réservoir, **caractérisée en ce que** l'on effectue les étapes suivantes:

- on calcule une grandeur D pour plusieurs échantillons de roche réservoir à partir de l'une au moins des grandeurs $S_{1r}$, $S_{2a}$, $S_{2b}$ et Q, et à partir de l'une au moins des formules suivantes:

$$D=S_{1r}/(S_{1r}+S_{2a}+Q),$$

$$D = S_{1r} + S_{2a})/(S_{1r} + S_{2a} + Q)$$

$$D=S_{2b}/(S_{1r}+S_{2a}),$$

- on mesure le degré API du pétrole produit par lesdites roches réservoirs desdits échantillons,
- on détermine une fonction approchée f reliant le degré API mesuré à D,
- on détermine une valeur approchée du degré API du pétrole en place dans une autre roche réservoir en prélevant au moins un échantillon de ladite autre roche, en calculant la grandeur D correspondante et en utilisant directement la fonction f.

7. Méthode selon l'une des revendications 1 à 5, dans laquelle ledit échantillon est une portion de pétrole produit, **caractérisée en ce que**:

- on calcule une grandeur D pour plusieurs échantillons provenant de différents réservoirs à partir de l'une au moins des grandeurs $S_{1r}$, $S_{2a}$, $S_{2b}$ et Q, et à partir de l'une au moins des formules suivantes:

$$D=S_{1r}/(S_{1r}+S_{2a}+Q),$$

$$D = (S_{1r} + S_{2a})/(S_{1r}+S_{2a} + Q)$$

$$D = S_{2b} / (S_{1r} + S_{2a}),$$

- on mesure le degré API desdits échantillons,
- on détermine une fonction approchée f1 reliant le degré API mesuré à D,
- on détermine une valeur approchée du degré API d'un pétrole d'une autre provenance, en calculant la grandeur D correspondante à un échantillon dudit pétrole d'une autre provenance et en utilisant directement la fonction f1.

8. Méthode selon l'une des revendications 6 et 7, **caractérisée en ce que** la fonction fl ou f a la forme suivante:

$$a \times 10^{be^{\alpha x}} + c$$

x étant le degré API ; a est compris entre 0,1 et 0,12; b est compris entre 1,7 et 2 ; c voisin de zéro ; α voisin de 2.

**Claims**

1. An improved method allowing fast assessment of at least one petroleum characteristic of a sample from geologic sediments, a method wherein said sample is heated in a nonoxidizing atmosphere according to several temperature rise stages, **characterized in that** said method comprises the stages as follows:

   a) the temperature of said sample is raised rapidly to a first temperature value below 200°C for a predetermined time,

   b) from the first temperature value, the temperature of the sample is raised to a second temperature value of about 370°C according to a temperature gradient ranging between 1 and 12°C/minute,

   c) the temperature of the sample is raised from the second value to a third temperature value at most equal to 800°C,

   d) three quantities $S_{1r}$, $S_{2a}$ and $S_{2b}$ representative of the amount of hydrocarbon-containing compounds contained in said sample are respectively determined in said stages a, b and c, quantity $S_{1r}$ being representative of the light hydrocarbons, such as those whose carbon number is less than about 15, $S_{2a}$ being representative of the heavier hydrocarbons, such as those whose carbon number ranges between about 15 and 40, and $S_{2b}$ being representative of the heavy hydrocarbon-containing compounds (resins, asphaltenes and/or kerogen), such as those whose carbon number is greater than about 40.

2. A method as claimed in claim 1, **characterized in that**, after raising the temperature to the third value, the residues of said sample are burned and a fourth value $R_C$ representative of the amount of residual organic carbon after pyrolysis is determined.

3. A method as claimed in any one of claims 1 to 2, **characterized in that** the amount Q of heavy products is assessed according to the formula as follows:

$$Q = S_{2b} + 10R_C/0.9.$$

(Q and S2b in milligram/gram).

4. A method as claimed in any one of claims 1 to 3, **characterized in that** certain hydrocarbons of the sample are distinguished by calculating a production index IP according to the formula as follows:

$$IP = (S_{1r} + S_{2a})/(S_{1r} + S_{2a} + S_{2b})$$

and **in that**, for IP > μ, μ ranging between 0 and 1, the sample contains resins and/or asphaltenes and, for IP < μ, the sample contains kerogen.

**5.** A method as claimed in claim 4, **characterized in that** a value around 0.4 is taken for $\mu$.

**6.** A method as claimed in any one of claims 1 to 5, wherein said sample is a piece of a reservoir rock, **characterized in that** the following stages are carried out:

- a quantity D is calculated for several reservoir rock samples from at least one of quantities $S_{1r}$, $S_{2a}$, $S_{2b}$ and Q, and from at least one of the following formulas:

$$D = S_{1r}/(S_{1r}+S_{2a}+Q),$$

$$D = (S_{1r} + S_{2a})/(S_{1r} + S_{2a} + Q),$$

$$D = S_{2b}/(S_{1r} + S_{2a}),$$

- the API degree of the oil produced by said reservoir rocks of said samples is measured,

- an approximate function f relating the measured API degree to D is determined,

- an approximate value of the API degree of the oil in place in another reservoir rock is determined by taking at least one sample of said other rock, by calculating the corresponding quantity D and by using directly function f.

**7.** A method as claimed in any one of claims 1 to 5, wherein said sample is a portion of produced petroleum, **characterized in that**:

- a quantity D is calculated for several samples coming from different reservoirs, from at least one of quantities $S_{1r}$, $S_{2a}$, S2b and Q, and from at least one of the following formulas:

$$D = S_{1r}/(S_{1r}+S_{2a}+Q),$$

$$D = (S_{1r} + S_{2a})/(s_{1r} + S_{2a} + Q),$$

$$D = S_{2b}/(S_{1r} + S_{2a}),$$

- the API degree of said samples is measured,

- an approximate function f1 relating the measured APT degree to D is determined,

- an approximate value of the API degree of a petroleum of another origin is determined by calculating the quantity D corresponding to a sample of said petroleum of another origin and by using directly function f1.

**8.** A method as claimed in any one of claims 6 to 7, wherein function f1 or f has the form as follows:

$$a \times 10^{be^{-\alpha x}} + c$$

x being the API degree; aranges between 0.11 and 0.12; b ranges between 1.7 and 2; c is close to zero and $\alpha$ is close to 2.

**Patentansprüche**

**1.** Verbessertes Verfahren zur schnellen Bewertung mindestens einer Erdöl-Eigenschaft einer Probe, die aus geologischen Ablagerungen (Sedimentgestein) stammt, bei dem die Probe in einer nicht-oxidierenden Atmosphäre in mehreren Temperaturerhöhungsstufen erwärmt wird, **dadurch gekennzeichnet, dass** das Verfahren die folgenden Stufen umfasst:

a) die Temperatur der Probe wird während einer vorgegebenen Zeitspanne schnell auf einen Temperaturwert von < 200 °C erhöht,
b) ausgehend von dem ersten Temperaturwert wird die Temperatur der Probe mit einem Temperaturgradienten zwischen 1 und 12 °C/min auf einen zweiten Temperaturwert von etwa 370 °C erhöht,
c) die Temperatur der Probe mit dem zweiten Temperaturwert wird auf einen dritten Temperaturwert von höchstens 800 °C erhöht,
d) im Verlaufe der genannten Stufen (a), (b) und (c) werden jeweils drei Größen $S_{1r}$, $S_{2a}$ und $S_{2b}$ bestimmt, die repräsentativ sind für die Menge der in der Probe enthaltenen Kohlenwasserstoff-Verbindungen, wobei die Größe $S_{1r}$ repräsentativ ist für leichte Kohlenwasserstoffe, z.B. solche, bei denen die Anzahl der Kohlenstoffatome weniger als etwa 15 beträgt, die Größe $S_{2a}$ repräsentativ ist für schwerere Kohlenwasserstoffe, z. B. solche, bei denen die Anzahl der Kohlenstoffatome zwischen etwa 15 und etwa 40 liegt, und die Größe $S_{2b}$ repräsentativ ist für schwere Kohlenwasserstoff-Verbindungen (Harze, Asphaltene und/oder Kerogen), z.B. solche, bei denen die Anzahl der Kohlenstoffatome mehr als 40 beträgt.

2. Verfahren nach Anspruch 1, **dadurch gekennzeichnet, dass** nach der Erhöhung der Temperatur auf den dritten Wert die Rückstände der Probe verbrannt werden und dass eine vierte Größe $R_c$ bestimmt wird, die repräsentativ ist für die Menge des nach der Pyrolyse zurückbleibenden organischen Kohlenstoffs.

3. Verfahren nach einem der Ansprüche 1 bis 2, **dadurch gekennzeichnet, dass** die Menge Q der schweren Produkte nach der folgenden Formel bewertet wird:

$$Q = S_{2b} + 10R_c/0,9 \text{ (Q und } S_{2b} \text{ sind angegeben in mg/g).}$$

4. Verfahren nach einem der Ansprüche 1 bis 3, **dadurch gekennzeichnet, dass** man bestimmte Kohlenwasserstoffe der Probe erkennt durch Berechnung eines Produktionsindex IP nach der folgenden Formel:

$$IP = (S_{1r} + S_{2a})/(S_{1r} + S_{2a} + S_{2b}) \text{ und}$$

dass für IP > μ, wobei μ zwischen 0 und 1 liegt, die Probe Harze und/oder Asphaltene enthält, und dass für IP < μ die Probe Kerogen (ein unlösliches organisches Material) enthält.

5. Verfahren nach Anspruch 4, **dadurch gekennzeichnet, dass** man für μ einen Wert in der Nähe von 0,4 annimmt.

6. Verfahren nach einem der Ansprüche 1 bis 5, bei dem die Probe ein Stück Erdölspeichergestein ist, **dadurch gekennzeichnet, dass** man die folgenden Stufen durchführt:

man berechnet eine Größe D für mehrere Erdölspeicher-Gesteinsproben, ausgehend von mindestens einer der Größen $S_{1r}$, $S_{2a}$, $S_{2b}$ und Q, nach mindestens einer der folgenden Formeln:

$$D = S_{1r}/(S_{1r} + S_{2a} + Q),$$

$$D = (S_{1r} + S_{2a})/(S_{1r} + S_{2a} + Q),$$

$$D = S_{2b}/(S_{1r} + S_{2a}),$$

- man bestimmt die API-Dichte (in Grad) des Erdöls, das aus den genannten Erdölspeicher-Gesteinsproben gewonnen wird,
- man bestimmt eine Näherungsfunktion f, welche die gemessene API-Dichte mit D verbindet, und
- man bestimmt einen Näherungswert für die API-Dichte des Erdöls, das in einem anderen Erdölspeichergestein enthalten ist, indem man mindestens eine Probe des anderen Gesteins entnimmt, bei dem man die entsprechende Größe D errechnet und direkt die Funktion f anwendet.

**7.** Verfahren nach einem der Ansprüche 1 bis 5, bei dem die genannte Probe ein Teil des gewonnenen Erdöls ist, **dadurch gekennzeichnet, dass** man

- eine Größe D für mehrere Proben errechnet, die aus verschiedenen Lagerstätten stammen, wobei man von mindestens einer der Größen $S_{1r}$, $S_{2a}$, $S_{2b}$ und Q ausgeht, nach mindestens einer der folgenden Formeln:

$$D = S_{1r}/(S_{1r} + S_{2a} + Q),$$

$$D = (S_{1r} + S_{2a})/(S_{1r} + S_{2a} + Q),$$

$$D = S_{2b}/(S_{1r} + S_{2a}),$$

- man bestimmt die API-Dichte der genannten Proben,
- man bestimmt eine Nährungsfunktion f1, welche die gemessene API-Dichte mit D verbindet, und
- man bestimmt einen Näherungswert für die API-Dichte eines Erdöls einer anderen Herkunft, indem man die entsprechende Größe D bei einer Probe des genannten Erdöls einer anderen Herkunft errechnet und direkt die Funktion f1 anwendet.

**8.** Verfahren nach einem der Ansprüche 6 bis 8, **dadurch gekennzeichnet, dass** die Funktion f1 oder f die folgende Formel hat:

$$a \times 10^{be^{-\alpha x}} + c$$

worin stehen x für die API-Dichte in Grad, a für einen Wert zwischen 0,11 und 0,12; b für einen Wert zwischen 1,7 und 2; c für einen Wert in der Nähe von 0 und $\alpha$ für einen Wert in der Nähe von 2.

## FIG.1

## FIG.2

## FIG.3

## FIG.5

FIG.4

## FIG.6

| 41 | 42 | 43 | 44 | 45 | 46 | 47 | 48 |
|----|----|----|----|----|----|----|----|
| DEPTH (meters) | S1r | S2a | NSO | KERO | T O C | P I | H I' |
| | LIGHT OIL | HEAVY OIL | RESINS + ASPHALTENS | KEROGEN QUANTITY | TOTAL ORGANIC CARBON | PRODUCTION INDEX | |

## FIG.7